(19) Europäisches Patentamt
European Patent Office
Office européen des brevets

(11) EP 0 402 228 B1

(12) FASCICULE DE BREVET EUROPEEN

(45) Date de publication et mention de la délivrance du brevet:
**03.01.1996 Bulletin 1996/01**

(21) Numéro de dépôt: **90401507.0**

(22) Date de dépôt: **05.06.1990**

(51) Int Cl.$^6$: **C07D 498/20**, C07D 513/20, G03C 1/685
// (C07D498/20, 265:00, 209:00, 209:00), (C07D513/20, 277:00, 265:00, 209:00)

# (54) Composés photochromiques de type indolino-spiro-oxazine à cycle à 5 chaînons, leur procédé de préparation, compositions et articles photochromiques contenant de tels composés

Fotochrome Indolino-spiro-oxazine mit einem fünfgliedrigen Ring, Verfahren zu ihrer Herstellung und diese Verbindungen enthaltende fotochrome Zusammensetzungen und Gegenstände

Indolino-spiro-oxazine photochromic compounds having a five-membered ring, process for their preparation and photochromic compositions and materials containing them

(84) Etats contractants désignés:
**CH DE ES FR GB IT LI**

(30) Priorité: **05.06.1989 FR 8907402**

(43) Date de publication de la demande:
**12.12.1990 Bulletin 1990/50**

(73) Titulaire:
**ESSILOR INTERNATIONAL (Compagnie Générale d'Optique)**
**F-94220 Charenton le Pont (FR)**

(72) Inventeurs:
- **Guglielmetti, Robert**
  **F-13009 Marseille (FR)**
- **Tardieu, Pascale**
  **F-13004 Marseille (FR)**

(74) Mandataire: **Thibon-Littaye, Annick et al**
**F-78160 Marly-le-Roi (FR)**

(56) Documents cités:
**EP-A- 0 245 020**
**EP-A- 0 313 941**
**EP-A- 0 358 774**
**WO-A-87/00524**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).



Printed by Jouve, 75001 PARIS (FR)

## Description

La présente invention concerne de nouveaux composés photochromiques du type indolino-spiro-oxazine, leur procédé d'obtention, ainsi que des compositions et articles à propriétés photochromiques renfermant au moins un desdits composés photochromiques.

Le photochromisme est un phénomène réversible bien connu, illustré par exemple par un composé qui change de couleur quand il est exposé à des radiations lumineuses dont certaines appartiennent au domaine de l'U.V., notamment les radiations solaires, et qui reprend sa couleur initiale lorsqu'on interrompt l'exposition lumineuse.

De tels composés sont utilisés par exemple dans la fabrication de lentilles pour lunettes de protection solaire ou dans d'autres applications où intervient le besoin de faire varier la transparence d'un article en fonction de l'intensité lumineuse environnante. Ils peuvent être appliqués sur un support transparent ou incorporés dans un matériau organique polymérisé transparent en combinaison avec une très grande variété de compositions polymériques.

Un certain nombre de composés photochromiques organiques comportant dans leur formule un groupe indolino-spiro-oxazine ont déjà été proposés pour de telles applications, notamment dans le domaine des lentilles de protection solaire.

Ainsi, les brevets américains US 3 562 172 et 3 578 602 ont divulgué l'effet photochromique de certains composés appartenant à la famille des indolino-spironaphtooxazines, tandis que le brevet américain US 4 215 010 décrit des indolinospironaphto-oxazines dont le cycle naphtalène est substitué par des groupements méthoxy, éthoxy ou un atome d'halogène. Des composés photochromiques analogues comportant un cycle pyridobenzène au lieu du cycle naphtalène des composés ci-dessus sont décrits dans le brevet américain US 4 720 547, ainsi que dans la demande internationale WO 87/00524.

Cependant, les composés décrits dans cet art antérieur ne satisfont pas pleinement à l'ensemble des qualités que l'on en attend. Si l'on se place plus particulièrement dans le cadre de l'application aux lentilles de protection solaire, le matériau photochromique obtenu, que ce soit à partir d'une solution du composé photochromique ou par incorporation de celui-ci dans un polymère organique, doit présenter un certain nombre de propriétés concernant l'effet photochromique, en plus de sa transparence naturelle et de sa compatibilité avec les matériaux couramment utilisés pour de telles lentilles.

En particulier :

- Quand il est irradié dans son domaine de photo-sensibilité, l'apparition de la coloration doit apparaître rapidement, en un temps de préférence de l'ordre de la seconde, et sa disparition quand l'irradiation cesse doit être tout aussi rapide.

- Le matériau doit être stable dans le temps, aussi bien par lui-même que dans son photochromisme. Ainsi, le composé doit pouvoir supporter au sein du matériau, un nombre élevé de cycles coloration-décoloration, tout au long d'une durée d'utilisation qui peut être de plusieurs années.

- La colorabilité doit être bonne pour des teneurs en composé photochromique raisonnables, et le spectre d'absorption du matériau irradié doit couvrir autant que possible l'ensemble du spectre visible.

- L'effet photochromique doit être, de préférence, indépendant du substrat renfermant le composé, et il doit se manifester dans une plage de températures étendue, tant à des températures ambiantes variables que quand il est échauffé sous irradiation.

- La coloration prise par le matériau sous irradiation doit aussi répondre à des soucis d'ordre esthétique, en préservant une vision de l'environnement agréable pour le porteur de lunettes ou lentilles ophtalmiques en de tels matériaux. De ce point de vue, il convient d'éviter les couleurs bleues auxquelles conduisent généralement les composés de l'art antérieur précité, et de privilégier le vert.

Dans le but de satisfaire mieux que les composés connus aux différents impératifs, l'invention propose des composés photochromiques, qui se caractérisent en ce qu'ils répondent à la formule d'une indolino-spiro-oxazine comportant une partie indolinique et une partie oxazine, où la partie oxazine comporte un hétérocycle insaturé à cinq chaînons qui comporte un atome de soufre et un atome d'azote comne dans le noyau thiazole, ledit hétérocycle comportant une liaison imine C=N-intracyclique, et en ce que ledit hétérocycle est ortho-condensé avec un noyau benzénique de ladite partie oxazine du composé, celle-ci étant du type benzo-oxazine, de sorte que la partie oxazine est benzothiazolo-oxazine, lesdits composés présentant la formule développée :

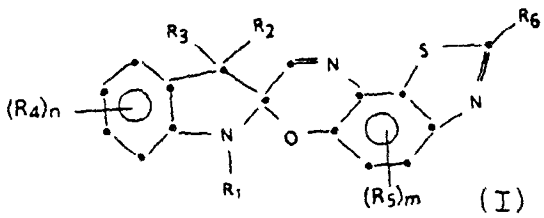

(I)

dans laquelle :

- n varie de 0 à 4,
- m prend les valeurs 1 ou 2,
- $R_1$ est un groupe $C_{1-4}$ alkyle tel que méthyle ou éthyle, isopropyle ou n-butyle ;
- $R_2$ et $R_3$ représentent chacun et indépendamment l'un de l'autre un groupe méthyle ou éthyle ;
- $R_4$ est un atome d'hydrogène, un groupe méthyle, méthoxy ou chloro ;
- $R_5$ est un atome d'hydrogène.
- et $R_6$ est un groupe méthyle.

La présente invention a également pour objet un procédé de préparation des composés indolino-spiro-oxazine photochromiques représentés par la formule (I).

Il exploite en seconde étape un schéma général de synthèse des indolino-spiro-oxazines qui est connu en lui-même et qui consiste à réaliser la condensation d'une base de Fischer ou composé alkylidène-2 indolinique sur un composé nitroso-hydroxy-hétéro-aromatique. En première étape, le composé nitroso-hydroxy-hétéro-aromatique convenable, tel que celui de type nitroso-7 hydroxy-6 benzothiazole par exemple, substitué éventuellement par un ou plusieurs groupes $R_5$, peut être préparé à partir d'un composé méthoxy approprié par hydrolyse en dérivé hydroxy correspondant, puis nitrosation de ce dernier.

Dans leur mise en oeuvre pour la préparation de compositions photochromiques, les composés selon la présente invention peuvent être dissous dans un solvant convenable tel que le toluène ou l'éthanol afin d'obtenir une solution photochromique. Ces mêmes composés photochromiques peuvent également être dissous au sein d'un polymère, d'un copolymère ou d'un mélange de polymères en solution dans un solvant organique convenable.

Ils entrent ainsi dans la constitution de compositions selon la présente invention, qui peuvent être appliquées ou introduites dans ou sur un matériau polymère organique transparent pour obtenir un article transparent photochromique. Préférentiellement, ce matériau est un matériau de qualité optique, plus particulièrement un matériau convenable pour la fabrication de lentilles ophtalmiques.

Ils peuvent aussi entrer dans des compositions photochromiques faisant également l'objet de la présente invention qui peuvent être utilisées directement dans la constitution de films plastiques photochromiques, de plaques et de lentilles telles que des lentilles pour lunettes de soleil, viseurs, optiques de caméra et filtres.

Des exemples de compositions appropriées, conformément à l'invention, pour la fabrication de matériaux et articles transparents photochromiques, comportent un ou plusieurs des composés photochromiques de la présente invention en combinaison avec un ou plusieurs des polymères suivants :
polymère d'un monomère de polyolallylcarbonate, poly-acrylate, polyalkyl-acrylates tels que le polyméthacrylate de méthyle (PMMA), acétate de cellulose, triacétate de cellulose, propiono et butyro-acétate de cellulose, acétate de polyvinyle, alcool polyvinylique, polyuréthanes, polycarbonates, polyéthylènetéréphtalate, polystyrène, copolymères de styrène et de méthacrylate de méthyle, acrylonitrile, polyvinylbutyral.

La quantité de composé photochromique (ou de composition contenant ce composé) appliquée ou introduite sur ou dans le matériau polymère n'est pas d'importance critique et elle dépend généralement de l'intensité de couleur désirée sous irradiation et de la méthode utilisée pour incorporer ou appliquer le composé photochromique. Cette dernière peut être choisie parmi les nombreuses méthodes applicables aux composés photochromiques de l'art antérieur, parmi lesquelles notamment la dissolution ou la dispersion du composé dans la composition de base du matériau, ou la réalisation d'une couche photochromique en surface ou à l'intérieur d'un matériau support transparent.

D'une manière générale, plus on ajoute de composé photochromique, plus la coloration sous irradiation sera importante. Une telle quantité peut être décrite comme une quantité photochromique. De façon usuelle, la quantité de composé photochromique incorporée au matériau optique est de 0,01 à 20 % en poids, et préférentiellement de 0,05 à 10 % en poids, par rapport au poids total de matériau optique.

On obtient ainsi des effets photochromiques qui se traduisent par l'apparition d'une coloration sous exposition à des radiations appartenant au domaine de l'U.V., avec retour à la couleur ou la transparence originelle lorsqu'on interrompt l'exposition aux radiations U.V. Ce changement de coloration peut se renouveler un très grand nombre de fois,

comme il est demandé pour des lunettes de protection solaire. De plus, la coloration persiste pendant tout le temps de l'exposition au rayonnement solaire mieux que dans le cas des composés photochromiques de l'art antérieur.

L'invention sera maintenant plus complètement illustrée au moyen d'exemples particuliers de mise en oeuvre non limitatifs.

EXEMPLE I

Etape 1

1) On mélange 1 g de méthoxy-6 méthyl-2 benzothiazole commercial ($5,5.10^{-3}$ moles) à 0,9 g d'acide bromhydrique azéotropique à 48 % ($1,1\ 10^{-2}$ mole). La réaction s'effectue en tube scellé à 125 °C durant 6 heures. Après neutralisation de la solution avec de l'ammoniaque 3N (pH=7), le composé hydroxylé est extrait au chloroforme. Le rendement est quantitatif.

Point de fusion : F = 147 °C ($C_8\ H_7$ N O S, M = 165).

2) On ajoute pendant une une heure, en agitant, une solution de $1,5\ 10^{-3}$ mole de nitrite de sodium dans 3 ml d'eau, à une solution refroidie (0-5 °C) de $1,5\ 10^{-3}$ mole de l'hydroxy-6 méthyl-2 benzothiazole obtenu précédemment, 0,3 ml d'acide chlorhydrique concentré et 3 ml d'eau distillée. On poursuit l'agitation pendant 1 h à basse température. On obtient ainsi l'hydroxy-6 nitroso-7 méthyl-2 benzothiazole sous la forme d'un précipité orange qui est lavé à l'eau puis séché.

Rendement 55 %, F = 215 °C ($C_8H_6N_2O_2S$, M = 194).

Etape 2

Le composé intermédiaire résultant de l'étape 1 est utilisé pour préparer un composé photochromique de formule I où $R_4$ est H et $R_1$, $R_2$, $R_3$ sont tous trois des groupes méthyle $CH_3$, comme suit :

$1,4\ 10^{-3}$ moles de base de Fischer triméthyl-1,3,3 méthylène-2 indoline sont dissous dans 10 ml de n-heptane et 2 ml d'éthanol anhydre et chauffés à reflux. A température constante (environ 76 °C), on ajoute lentement, en 2 h, $1,4\ 10^{-3}$ moles du réactif nitroso hydroxy hétérocyclique de l'étape I en suspension dans 12 ml d'éthanol anhydre.

Le mélange réactionnel est maintenu à reflux pendant 45 mn. Tandis que la réaction progresse lentement, on élimine l'eau avec l'éthanol par un dispositif de Dean Stark. On ajoute ensuite environ 30 ml d'éthanol pour conserver un volume constant en solvant.

Après avoir fait passer le mélange à travers une colonne chromatographique de gel de silice et éliminé le solvant par évaporation, on recristallise le résidu solide dans un mélange approprié de solvants, généralement apolaires tels que éther de pétrole, diéthyl-éther, n-hexane ou benzène. Mais on peut également utiliser le méthanol ou le toluène.

Le produit obtenu est constitué par l'indolino-spiro-oxazine de formule I, où $R_6$ est $CH_3$, soit la triméthylindolino-spiro-méthyl-2 benzothiazolo-oxazine. Le rendement est de l'ordre de 60 % quand le solvant réactionnel est un mélange de n-heptane et éthanol. Une purification ultérieure par recristallisation en milieu solvant ramène à 40 % le rendement final.

EXEMPLE II

Sous atmosphère inerte d'azote ou d'argon, on ajoute 1,4 ml d'une solution 1M de $BBr_3$ dans le dichloro-méthane à une solution agitée à 0 °C de $1,2\ 10^{-3}$ moles de méthoxy-5 triméthyl-2,3,3 indolénine dans 1,5 ml de dichlorométhane, pour obtenir l'hydroxy-5 triméthyl-2,3,3 indolénine.

Le solution est agitée à la température ambiante et un précipité apparaît. A la fin de la réaction, on effectue une hydrolyse avec HCl 0,1N.

On procède alors à une nitrosation conformément au mode opératoire du 2) de l'étape 1 de l'exemple I, de manière à obtenir la nitroso-4 hydroxy-5 triméthyl-2,3,3 indolénine.

En adoptant la même procédure que dans l'étape 2 de l'exemple I, et en utilisant la même base de Fischer indolinique, on soumet le composé précédent au traitement de condensation spirannique.

On obtient ainsi la triméthylindolino-spiro-indolénino-oxazine, avec un rendement de l'ordre de 40 %.

EXEMPLE III

Par la procédure de l'exemple I, on prépare le composé diméthyl isopropyl indolino-spiro-méthyl-2 benzothiazolo-oxazine correspondant à la formule I où $R_2$ et $R_3$, $R_6$ sont des groupes méthyle, $R_1$ est un groupe isopropyle et $R_4$ et $R_5$ sont des atomes d'hydrogène.

EXEMPLE IV

Par la procédure de l'exemple I, on prépare le composé triméthyl méthoxy-5 indolino-spiro-méthyl-2 benzothiazolo-oxazine correspondant à la formule I où $R_1$, $R_2$,$R_3$ et $R_6$ sont des groupes méthyle, $R_4$ est un groupe méthoxy en position 5 du cycle indoline et $R_5$ est un atome d'hydrogène.

EXEMPLE V

Les composés photochromiques obtenus dans les exemples précédents sont utilisés dans la préparation de vernis à base polysiloxanes du type de ceux décrits dans le brevet français FR 82 0440, à raison de 1 % en poids. Le vernis obtenu est appliqué sur des lentilles ophtalmiques en matériau organique, puis durcis pendant 2 h à 100 °C. L'épaisseur des couches obtenues est de 2 microns. Le pourcentage en poids de composé photochromique dans la couche durcie est de l'ordre de 5 %.

EXEMPLE VI

Les différents composés des exemples précédents, en solution dans un solvant, à la même concentration dans les différents échantillons, sont soumis à des essais de coloration sous irradiation, en comparaison avec des composés photochromiques de l'art antérieur. Pour chaque échantillon, on détermine la longueur d'onde correspondant au maximum de son domaine de photocoloration (λmax) ou les limites du pic de photocoloration.

Les résultats obtenus sont consignés ci-après :

| Composé<br>Art antérieur : | λ max (nm) | Couleur |
|---|---|---|
| Triméthylindolino-spiro-benzo-oxazine | 590 | Bleu |
| Homologue N-méthoxyéthyl | 606 | Bleu |
| Homologue 6-méthoxy N-méthoxyéthyl | 603 | Bleu |

| Composés de l'invention : | Pic (nm) | Couleur |
|---|---|---|
| Exemple I | 621 | Bleu vert |
| Exemple III | 626 | Bleu vert |
| Exempe IV | 632 | Bleu vert |

On observe donc, grâce à l'invention, un déplacement dans le vert de la coloration apparaissant sous irradiation, ce qui est préférable pour des composés destinés à des lunettes ou lentilles de protection solaire.

**Revendications**

**Revendications pour les Etats contractants suivants : CH, DE, FR, GB, IT, LI**

1. Composés photochromiques, caractérisés en ce qu'ils répondent à la formule d'une indolinospiro-oxazine comportant une partie indolinique et une partie oxazine, où la partie oxazine comporte un hétérocycle insaturé à cinq chaînons ortho-condensé avec un noyau benzénique et comportant une liaison imine C=N- intracyclique et un hétéroatome de soufre, de sorte que lesdits composés sont des benzothiazolo-oxazines présentant la formule développée suivante :

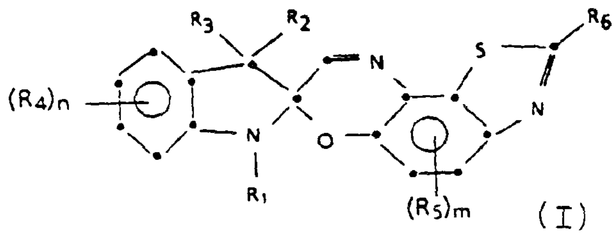

(I)

dans laquelle :

- n varie de 0 à 4,
- m prend les valeurs 1 ou 2,
- $R_1$ est un groupe $C_{1-4}$ alkyle tel que méthyle ou éthyle, isopropyle ou n-butyle ;
- $R_2$ et $R_3$ représentent chacun et indépendamment l'un de l'autre un groupe méthyle ou éthyle ;
- $R_4$ est un atome d'hydrogène, un groupe méthyle, méthoxy ou chloro ;
- $R_5$ est un atome d'hydrogène.
- et $R_6$ est un groupe méthyle.

**2.** Composés photochromiques suivant la revendication 1, caractérisés en ce que :

- $R_1$ est un groupe méthyle ou isopropyle,
- $R_2$ et $R_3$ sont chacun un groupe méthyle,
- $R_4$ est l'hydrogène ou un groupe méthoxy,
- $R_5$ est un atome d'hydrogène,
- et $R_6$ est un groupe méthyle.

**3.** Procédé de préparation des composés selon l'une quelconque des revendications 1 à 2, caractérisé en ce qu'il comporte la condensation d'une base de Fischer ou composé alkylidène-2 indolinique sur un composé nitroso-hydroxy-hétéro aromatique (notamment benzothiazole, substitué éventuellement par un ou plusieurs groupes $R_5$ et $R_6$ tels que définis pour les formules I ) obtenu préalablement à partir d'un composé méthoxy approprié par hydrolyse en dérivé hydroxy correspondant, puis nitrosation de ce dernier.

**4.** Compositions et articles photochromiques sous la lumière solaire, contenant au moins un composé selon l'une quelconque des revendications 1 à 3, en proportion de 0,01 à 20 % en poids, et de préférence de 0,05 à 10 % en poids.

**5.** Composition suivant la revendication 4, caractérisée en ce qu'elle constitue un vernis photochromique à base de polysiloxanes pour lentilles de protection solaire.

**Revendications pour l'Etat contractant suivant : ES**

**1.** Procédé de préparation de composés photochromiques, caractérisé en ce qu'il comporte la condensation d'une base de Fischer ou composé alkylidène-2 indolinique sur un composé nitroso-hydroxy- hétéroaromatique (notamment benzothiazole, substitué éventuellement par un hydrogène ou un groupement méthyle) de manière à obtenir un composé répondant à la formule développée suivante :

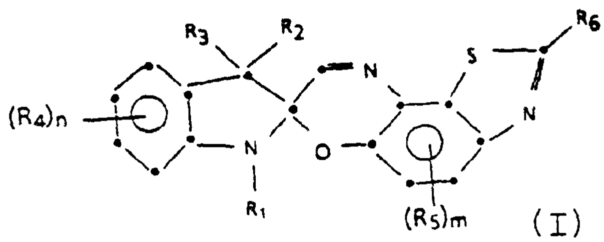

(I)

dans laquelle :

- n varie de 0 à 4 ;
- m prend les valeurs 1 ou 2 ;
- $R_1$ représente un groupe $C_{1-4}$ alkyle tel que méthyle, éthyle, isopropyle, n-butyle ;
- $R_2$ et $R_3$ représentent chacun et indépendamment l'un de l'autre un groupe méthyle ou éthyle ;
- $R_4$ est un atome d'hydrogène, un groupe méthyle, méthoxy ou chloro ;
- et $R_5$ est un atome d'hydrogène ;
- et $R_6$ est un groupe méthyle.

**2.** Procédé suivant la revendication 1, caractérisé en ce que ledit composé nitroso-hydroxy-hétéro-aromatique est obtenu préalablement à partir d'un composé méthoxy approprié par hydrolyse en dérivé hydroxy correspondant, puis nitrosation de ce dernier.

**3.** Procédé suivant la revendication 1, caractérisé en ce que :

- $R_1$ est un groupe méthyle ou isopropyle ;
- $R_2$ et $R_3$ sont chacun un groupe méthyle ;
- $R_4$ est l'hydrogène ou un groupe méthoxy ;
- $R_5$ est un atome d'hydrogène ;
- et $R_6$ est un groupe méthyle.

**4.** Procédé de préparation d'une composition photochromique pour lentilles de protection solaire, caractérisé en ce que ladite composition contient au moins un composé répondant à la formule suivant l'une quelconque des revendications 1 et 2.

**5.** Procédé suivant la revendication 4, caractérisé en ce que ladite composition est un vernis photochromique de type polysiloxane.

**6.** Procédé suivant la revendication 4 ou 5, caractérisé en ce que ledit composé est en proportion de 0,01 % à 20 % en poids, et de préférence de 0,05 à 10 % en poids dans ladite composition.

**Patentansprüche**

**Patentansprüche für folgende Vertragsstaaten : CH, DE, FR, GB, IT, LI**

**1.** Photochrome Verbindungen, dadurch gekennzeichnet, daß sie der Formel eines Indolinospiro-oxazins entsprechen, die einen Indolinanteil und einen Oxazolinanteil umfaßt, wobei der Oxazinanteil einen ungesättigten fünfgliedrigen Heterocyclus umfaßt, der in Orthostellung mit einem Benzolring kondensiert ist und eine intracyclische Iminbindung und ein Schwefel-Heteroatom aufweist, so daß die Verbindungen Benzothiazolo-oxazine mit der unten angegebenen Formel sind:

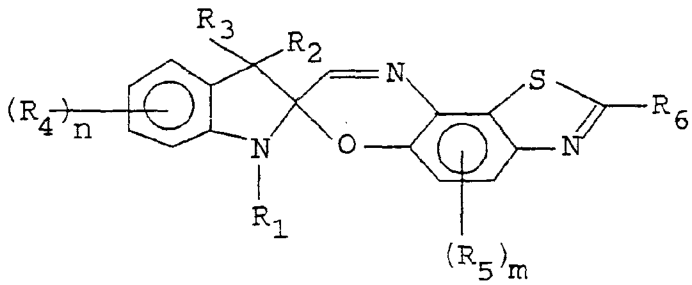

(I)

in der

- n von 0 bis 4 variiert,
- m die Werte 1 oder 2 annimmt,
- $R_1$ eine $C_1$- bis $C_4$-Alkylgruppe wie Methyl oder Ethyl oder Isopropyl oder n-Butyl ist,
- $R_2$ und $R_3$ jeweils und unabhängig voneinander eine Methylgruppe oder Ethylgruppe wiedergeben,
- $R_4$ ein Wasserstoffatom, eine Methylgruppe, Methoxygruppe oder ein Chloratom ist, und
- $R_5$ ein Wasserstoffatom ist, und
- $R_6$ eine Methylgruppe ist.

**2.** Photochrome Verbindungen nach Anspruch 1, dadurch gekennzeichnet, daß

- $R_1$ eine Methylgruppe oder Isopropylgruppe ist,
- $R_2$ und $R_3$ jeweils eine Methylgruppe sind,
- $R_4$ ein Wasserstoffatom oder eine Methoxygruppe ist,
- $R_5$ ein Wasserstoffatom ist, und
- $R_6$ eine Methylgruppe ist.

**3.** Verfahren zur Herstellung von Verbindungen gemäß einem der Ansprüche 1 bis 2, dadurch gekennzeichnet, daß es eine Kondensation einer Fischerbase oder 2-Alkylidenindolinverbindung mit einer nitroso-hydroxy-heteroaromatischen Verbindung (insbesondere Benzothiazol, das gegebenenfalls mit einer oder mehreren Gruppen $R_5$ und $R_6$ wie in der Formel I definiert substituiert ist) umfaßt, welche zuvor ausgehend von einer geeigneten Methoxyverbindung durch Hydrolyse zum entsprechenden Hydroxyderivat und nachfolgender Nitrosierung der Letzteren erhalten wurde.

**4.** Unter Einwirkung von Sonnenlicht photochrome Zusammensetzungen und Gegenstände, die mindestens eine Verbindung gemäß einem der Ansprüche 1 bis 3 in einem Anteil von 0,01 bis 20 Gew.% und vorzugsweise 0,05 bis 10 Gew.% enthalten.

**5.** Zusammensetzung nach Anspruch 4, dadurch gekennzeichnet, daß sie einen photochromen Lack auf Polysiloxanbasis für Sonnenschutzlinsen bildet.

**Patentansprüche für folgenden Vertragsstaat : ES**

**1.** Verfahren zur Herstellung von photochromen Verbindungen, dadurch gekennzeichnet, daß eine Fischerbase oder 2-Alkyliden-indolinverbindung mit einer Nitroso-hydroxy-heteroaromatischen Verbindung (insbesondere Benzothiazol, das gegebenenfalls mit einem Wasserstoffatom oder einer Methylgruppe substituiert ist) in einer Weise kondensiert wird, um eine Verbindung mit der nachfolgend angegebenen Formel zu erhalten:

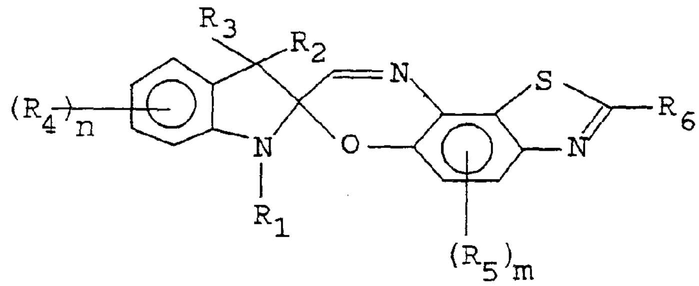

(I)

in der

- n von 0 bis 4 variiert,
- m die Werte 1 oder 2 annimmt,
- $R_1$ eine $C_1$- bis $C_4$-Alkylgruppe wie Methyl, Ethyl, Isopropyl, n-Butyl wiedergibt,
- $R_1$ und $R_3$ jeweils und unabhängig voneinander eine Methylgruppe oder Ethylgruppe wiedergeben,
- $R_4$ ein Wasserstoffatom, eine Methylgruppe, Methoxygruppe oder ein Chloratom ist, und
- $R_5$ ein Wasserstoffatom ist, und
- $R_6$ eine Methylgruppe ist.

2. Verfahren nach Anspruch 1, dadurch gekennzeichnet, daß die nitroso-hydroxy-heteroaromatische Verbindung zuvor ausgehend von einer geeigneten Methoxyverbindung durch Hydrolyse zum entsprechenden Hydroxyderivat und nachfolgende Nitrosierung des Letzteren erhalten worden ist.

3. Verfahren nach Anspruch 1, dadurch gekennzeichnet, daß

- $R_1$ eine Methylgruppe oder Isopropylgruppe ist,
- $R_2$ und $R_3$ jeweils eine Methylgruppe sind,
- $R_4$ ein Wasserstoffatom oder eine Methoxygruppe ist,
- $R_5$ ein Wasserstoffatom ist, und
- $R_6$eine Methylgruppe ist.

4. Verfahren zur Herstellung einer photochromen Zusammensetzung für Sonnenschutzlinsen, dadurch gekennzeichnet, daß die Zusammensetzung mindestens eine Verbindung mit der Formel gemäß einem der Ansprüche 1 und 2 enthält.

5. Verfahren nach Anspruch 4, dadurch gekennzeichnet, daß die Zusammensetzung ein photochromer Lack vom Polysiloxantyp ist.

6. Verfahren nach Anspruch 4 oder 5, dadurch gekennzeichnet, daß die Verbindung in einem Anteil von 0,01 bis 20 Gew.% und vorzugsweise 0,05 bis 10 Gew.% in der Zusammensetzung enthalten ist.

**Claims**

**Claims for the following Contracting States : CH, DE, FR, GB, IT, LI**

1. Photochromic compounds characterized in that they have the formula of an indolino-spiro-oxazine comprising an indolinic part and an oxazine part wherein said oxazine part comprises an unsaturated orthocondensed five-links heterocycle with a benzenic ring and having an intracyclic imine C=N link and a sulphur heteroatom, such that said compounds are benzothiazolo-oxazines having the following developed formula:

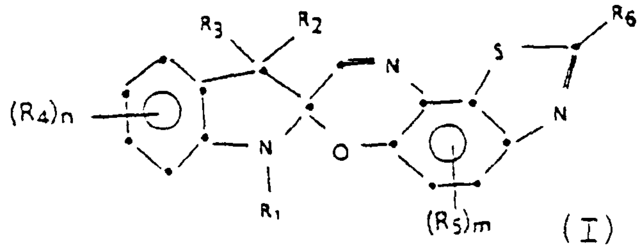

(I)

in which :

- n varies from 0 to 4;
- m can take values 1 or 2;
- $R_1$ is a $C_{1-4}$ alkyl group, such as a methyl, ethyl, isopropyl or n-butyl group;
- $R_2$ and $R_3$ each indepedently represent a methyl, or ethyl group;
- $R_4$ is a hydrogen atom, or a methyl, methoxy, or chloro group;
- $R_5$ is a hydrogen atom; and
- $R_6$ is a methyl group.

**2.** Photochromic compounds according to claim I characterized in that:

- $R_1$ is a methyl, or isopropyl group;
- $R_2$ and $R_3$ each are a methyl group;
- $R_4$ is hydrogen or a methoxy group;
- $R_5$ is a hydrogen atom;
- and $R_6$ is a methyl group.

**3.** A method for preparing photochromic compounds according to any of claims 1 or 2, characterized in that it comprises condensing a Fischer base, or a 2-alkylidene indolinic compound with a nitroso-hydroxy hetero aromatic compound (in particular benzothiazole optionally substituted by one or several $R_5$ and $R_6$ groups such as specified for the formula I) previously prepared from a convenient methoxy compound by hydrolising it into a corresponding hydroxy derivative, then nitrozating the latter.

**4.** Compositions and articles which are photochromic under sunlight, comprising at least a compound according to any of Claims 1 to 3 in the proportion from 0,01 to 20 % by weight, and preferably from 0,05 to 10 % by weight.

**5.** Composition according to Claim 4, characterized in that it constitutes a polysiloxane based photochromic varnish for sunlight protection lenses.

**Claims for the following Contracting State : ES**

**1.** A method for preparing photochromic compounds characterized in that it comprises condensing a Fischer base or 2-alkylidene indolinic compound with a nitroso-hydroxy hetero aromatic compound (in particular benzothiazole optionally substituted by a hydrogen or a methyl group) so as to obtain a compounds having the following developed formula:

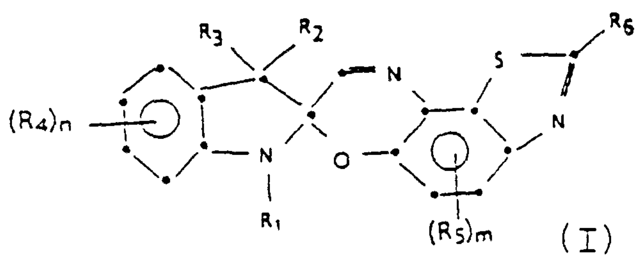

(I)

in which :

- n varies from 0 to 4;
- m can take values 1 or 2;
- $R_1$ is a $C_{1-4}$ alkyl group, such as a methyl, ethyl, isopropyl or n-butyl group;
- $R_2$ and $R_3$ each indepedently represent a methyl, or ethyl group;
- $R_4$ is a hydrogen atom, or a methyl, methoxy, or chloro group;
- $R_5$ is a hydrogen atom; and
- and $R_6$ is a methyl group.

**2.** A method according to claim 1, characterized in that the nitroso-hydroxy hetero aromatic compound is previously obtained from a convenient methoxy compound by hydrolising it into a corresponding hydroxy derivative, then nitrozating the latter.

**3.** A method according to claim 1, characterized in that

- $R_1$ is a methyl or an isopropyl group;
- $R_2$ and $R_3$ each are a methyl group;
- $R_4$ is a hydrogen atom or a methoxy group;
- $R_5$ is a hydrogen atom;
- and $R_6$ is a methyl group.

**4.** A method of preparing a photochromic composition pour sunlight protection lenses, characterized in that said composition contains at least one of the compounds defined by the formula according to any of claims 1 and 2.

**5.** A method according to Claim 4, characterized in that said composition is a polysiloxane based photochromic varnish.

**6.** A method according to Claim 4 or 5, characterized in that said compound is in a proportion from 0,01 to 20 % by weight, and preferably from 0,05 to 10 % by weight in said composition.